# EUROPEAN PATENT SPECIFICATION

(11) **EP 2 741 084 B1**
(45) Date of publication and mention of the grant of the patent: **22.01.2020**
(21) Application number: 12196171.8
(22) Date of filing: 07.12.2012
(51) Int. Cl.: G01N 33/574

(54) **Method of prognosing the clinical course of chronic lymphocytic leukemia (CLL)**
Verfahren zur Vorhersage des klinischen Verlaufs von chronischer lymphozytischer Leukämie (CLL)
Procédé de pronostic de l'évolution clinique de la leucémie lymphocytaire chronique (LLC)

(43) Date of publication of application: 11.06.2014
(73) Proprietor: AVA Lifescience GmbH, 79211 Denzlingen (DE)
(72) Inventor: Jumaa, Hassan, 79189 Bad Krozingen (DE)
(74) Representative: Stürken, Joachim

(56) References cited:
- GORDON J ET AL: "Phenotypic modulation of chronic lymphocytic leukemia cells by phorbol ester: induction of IgM secretion and changes in the expression of B cell-associated surface antigens.", JOURNAL OF IMMUNOLOGY (BALTIMORE, MD. : 1950) JAN 1984, vol. 132, no. 1, January 1984 (1984-01), pages 541-547, XP002693241, ISSN: 0022-1767
- KIMBY E ET AL: "Surface immunoglobulin pattern of the leukaemic cell population in chronic lymphocytic leukaemia (CLL) in relation to disease activity.", HEMATOLOGICAL ONCOLOGY 1985 OCT-DEC, vol. 3, no. 4, October 1985 (1985-10), pages 261-269, XP002693242, ISSN: 0278-0232
- YIN C CAMERON ET AL: "Chronic lymphocytic leukemia/small lymphocytic lymphoma associated with IgM paraprotein.", AMERICAN JOURNAL OF CLINICAL PATHOLOGY APR 2005, vol. 123, no. 4, April 2005 (2005-04), pages 594-602, XP002693243, ISSN: 0002-9173
- VILPO JUHANI ET AL: "Surface antigen expression and correlation with variable heavy-chain gene mutation status in chronic lymphocytic leukemia.", EUROPEAN JOURNAL OF HAEMATOLOGY JAN 2003, vol. 70, no. 1, January 2003 (2003-01), pages 53-59, XP002693244, ISSN: 0902-4441
- POTTER KATHLEEN N ET AL: "Structural and functional features of the B-cell receptor in IgG-positive chronic lymphocytic leukemia.", CLINICAL CANCER RESEARCH : AN OFFICIAL JOURNAL OF THE AMERICAN ASSOCIATION FOR CANCER RESEARCH 15 MAR 2006, vol. 12, no. 6, 15 March 2006 (2006-03-15) , pages 1672-1679, XP002693245, ISSN: 1078-0432
- CALPE EVA ET AL: "ZAP-70 enhances migration of malignant B lymphocytes toward CCL21 by inducing CCR7 expression via IgM-ERK1/2 activation.", BLOOD 20 OCT 2011, vol. 118, no. 16, 20 October 2011 (2011-10-20), pages 4401-4410, XP002693246, ISSN: 1528-0020
- Schriever F ET AL: "New Directions in the Diagnosis and Treatment of Chronic Lymphocytic Leukaemia", Drugs, 1 January 2003 (2003-01-01), pages 953-969, XP055430067, Cham DOI: 10.2165/00003495-200363100-00003 Retrieved from the Internet: URL:file:///C:\Users\EM51949\AppData\Roami ng\Mozilla\Firefox\Profiles\EM51949\CiteNP LTemp\CiteNPLWebPage.pdf [retrieved on 2017-11-29]

## Description

The present invention relates to a method of prognosing whether a patient afflicted with B-cell chronic lymphocytic leukemia (B-CLL) develops an aggressive or an indolent clinical course of B-CLL comprising determining the expression levels of IgD and IgM in a sample obtained from said patient and employing the ratio of IgD and IgM expression levels as an indicator for predicting whether said patient develops an aggressive or an indolent clinical course of B-CLL. In the method of the invention, a ratio of an expression level of IgD vs. IgM ≤ 1.15 is indicative of the patient developing an aggressive clinical course of B-CLL, and a ratio of an expression level of IgD vs. IgM > 1.15 is indicative of the patient developing an indolent clinical course of B-CLL.

In this specification, a number of documents including patent applications and manufacturer's manuals are cited.

B cell development can be divided into multiple stages according to the rearrangement status of the immunoglobulin (Ig) heavy (HC) and light (LC) chain genes (Rajewsky, K., Nature 381, 751-758 (1996)). Productive recombination of the HC gene leads to the expression of precursor-B cell receptors (pre-BCR) consisting of µHC together with the surrogate LC (λ5 and VpreB) and the signal-transduction heterodimer Ig-α/β (Herzog, S., Reth. M. & Jumaa, H., Nat Rev Immunol 9, 195-205 (2009)). The pre-BCR induces ligand-independent cell autonomous signalling that results in proliferation and Ig LC gene recombination (Ohnishi, K. & Melchers. F., Nat Immunol 4, 849-856 (2003); Meixlsperger, S., et al., Immunity 26, 323-333 (2007); Ubelhart, R., et al., Nat Immunol 11, 759-765 (2010). It has recently been shown that autoreactive BCRs activate cell-autonomous signalling and are able to replace pre-BCR function in B cell development (Eschbach, C., et al., Eur J Immunol 41. 2397-2403 (2011): Kohler, F., et al., Immunity 29, 912-921 (2008)). Since CLL-derived BCRs are also largely autoreactive (Herve, M., et al., J Clin Invest 115, 1636-1643 (2005); Sthoeger, Z.M., et al., J Exp Med 169, 255-268 (1989)), it is conceivable that they might mimic pre-BCR function and activate ligand-independent autonomous signalling.

CLL is one of the most frequent B cell malignancies in western countries (Morton, L.M., et al., Blood 107, 265-276 (2006)). CLL cases can be divided into two groups based on the mutational status of the Ig genes. CLL patients with leukemia cells that express mutated BCRs (M-CLL) have a better prognosis than CLL patients with unmutated BCRs (UM-CLL) (Rassenti, L.Z., et al., N Engl J Med 351, 893-901 (2004); Hamblin, T.J., Davis, Z., Gardiner, A., Oscier, D.G. & Stevenson, F.K., Blood 94, 1848-1854 (1999); Damle, R.N., et al., Blood 94, 1840-1847 (1999)). Yet, a prognosis solely on the basis of classifying the mutational status is not satisfying since about 25% of the patients show a similar clinical course of disease irrespective of the mutational status. Little is known about the origin of the two CLL subgroups. However, since somatic hypermutation takes place in germinal centres (GC), it has been proposed that UM-CLL B cells originate from pre-GC B cells, whereas the precursors for M-CLL B may be found among post-GC B cells (Klein, U., et al., Proc Natl Acad Sci U S A 100, 2639-2644 (2003)). Nevertheless, UM- and M-CLLs display similar gene expression profiles indicating common mechanisms that activate cell expansion (Rosenwald, A., et al., J Exp Med 194, 1639-1647 (2001); Klein, U., et al., J Exp Med 194, 1625-1638 (2001)).

Remarkably, B cells from around 1% of both UM-CLL and M-CLL cases express almost identical BCRs. In 20% of the cases the BCRs can be classified into certain stereotypes according to structural similarities and HC complementarity determining region 3 (HCDR3) (Ghiotto, F., et al., J Clin Invest 113, 1008-1016 (2004); Stamatopoulos, K., et al., Blood 109, 259-270 (2007); Widhopf, G.F., 2nd, et al., Blood 104, 2499-2504 (2004)). This restricted BCR repertoire has been interpreted as the result of a BCR-driven selection mechanism initiated by specific antigens that may promote the expansion of a respective CLL clone (Ghia, P., Chiorazzi, N. & Stamatopoulos, K., J Intern Med 264, 549-562 (2008); Stevenson, F.K., Krysov, S., Davies, A.J., Steele, A.J. & Packham, G., Blood 118, 4313-4320 (2011)). Although the antigens recognized by CLL BCRs remain to be identified, they may include autoantigens, as substantial numbers of CLL-derived antibodies have been reported to be autoreactive (Herve, M., et al., J Clin Invest 115, 1636-1643 (2005); Sthoeger, Z.M., et al., J Exp Med 169, 255-268 (1989)).

Presently, the diagnosis of CLL is typically based on a complete blood count (CBC) test, wherein an increase in the numbers of lymphocytes, which are infection-fighting immune cells, to at least 5000/µl, which is maintained over a time span of at least four weeks, is indicative of CLL. The diagnosis is usually confirmed and refined in clinical laboratories by FACS analysis of blood samples using commercially available antibodies against different surface markers. In this analysis CLL B cells are identified by the following characteristics: In CLL B cells, the CD5 antigen is expressed together with B cell markers such as CD19, CD20 and CD23. Characteristically, surface immunoglobulin, CD79b, CD20 and CD22 are only weakly expressed. In addition, CLL B cells are monoclonal with regard to the type of immunoglobulin light chain that is expressed (either kappa or lambda).

State of the art in the prediction of the clinical course of a CLL is an analysis of the mutational status of the BCRs expressed on the leukemic cells by PCR and subsequent sequencing of the amplified DNA molecules. This analysis is often combined with an analysis of diagnostic markers such as ZAP70 and CD38 or an examination for chromosomal aberrations. In addition, kits based on microarray expression analysis are commercially available such as the Vysis CLL FISH Probe Kit. WO2005/034733 describes the use of specific stereotypical CLL receptors and of derivatives thereof for the diagnosis and therapy of CLL subgroups.

There still remains the need for an inexpensive test for the prediction of the clinical course of a CLL with a sufficient probability of a correct prediction that can be performed easily and in a short amount of time. Solving this problem, i.e. being able to predict whether a patient afflicted with CLL will likely develop an aggressive or an indolent (non-aggressive) form of CLL is expected to assist the physician treating the patient in the selection of appropriate counter-measures or cures, in particular at early such as non-symptomatic stages of the disease. This need is addressed by the present invention.

The present invention is defined by the appended claims.

Accordingly, the present invention relates to a method of prognosing whether a patient afflicted with B-cell chronic lymphocytic leukemia (B-CLL) develops an aggressive or an indolent clinical course of B-CLL comprising determining the expression levels of IgD and IgM in a sample obtained from said patient and employing the ratio of IgD and IgM expression levels as an indicator for predicting whether said patient develops an aggressive or an indolent clinical course of B-CLL.

The following terms are all known in the art and are used in accordance with the definitions provided there. The definitions provided in this specification are solely intended to reflect the understanding of these terms in the art. Where nevertheless a discrepancy arises, the definitions provided herein supersede.

The term "prognosing", is intended to mean the prediction of the likely course and/or outcome of a disease, in the present case CLL. The prognosis optionally involves a detailed description of the course of the disease. Prognosing may also include the prediction of the likelihood of recovery from the disease. The term may also include the prediction of response to medication. Medication currently employed in treating CLL includes chemotherapeutics, such as Fludarabin, Cyclophosphamid, Leustatin, Bendamustin or Chlorambucil. In addition, monoclonal antibodies such as Rituximab, Arzerra (α-CD20) or, in patients with p53 deletions, Alemtuzumab (α-CD52) may be used. The standard treatment consists of a combination of Fludarabin, Cyclophosphamid and Rituximab which is referred to as "FCR-scheme".

The term "chronic lymphocytic leukemia (CLL)" is intended to mean a monoclonal disorder characterized by a progressive accumulation of functionally incompetent lymphocytes. It is the most common form of leukemia found in adults in western countries (Elter et al. (2006), Expert Opin Pharmacother, 7(12):1641-51). CLL mainly affects the development of B cell lymphocytes. Under normal conditions B cell lymphocytes produce immunoglobulins (also called antibodies) that help protect bodies of animals or humans against infection and disease. In individuals having CLL, lymphocytes undergo a malignant (cancerous) change and become leukemic cells.

In general, leukemia is a type of cancer of the blood or bone marrow characterized by an abnormal increase of immature white blood cells also called "blasts" or leukocytes. Chronic leukemia is characterized by the excessive build up of relatively mature, but still abnormal, white blood cells. Typically taking months or years to progress, the cells are produced at a much higher rate than normal, resulting in many abnormal white blood cells. In lymphocytic leukemias the cancerous change takes place in a type of marrow cell that normally goes on to form lymphocytes. Most lymphocytic leukemias involve a specific subtype of lymphocytes, the B cells. CLL B cells are characterized by co-expression of the CD5 antigen with B cell markers such as CD19, CD20 and CD23 accompanied by low level expression of surface immunoglobulin, CD79b, CD20 and CD22. These cells express either kappa or lambda immunoglobulin light chains.

The term "aggressive clinical course of CLL" is intended to mean a course of the disease with relatively rapid progression of the disease including e.g. short time to first treatment, short progression-free survival, poor or no response to treatment, high risk of (rapid) recurrence after treatment, low survival rates and/or low survival time.

The term "indolent clinical course of CLL" is intended to mean a course of the disease with relatively slow or no progression of the disease including e.g. long time to first treatment, long progression-free survival, good response to treatment, low risk of recurrence after treatment, high survival rates and/or high survival time.

The terms "IgD" which is an abbreviation of immunoglobulin D and "IgM" which is an abbreviation of immunoglobulin M, each designate a certain type of antibody (or immunoglobulin). IgD mainly functions as an antigen receptor on B cells that have not been exposed to antigens (Geisberger et al. (2006), Immunology; 118(4):429-437). IgD has been shown to activate basophils and mast cells and to effect the production of antimicrobial factors (Chen et al. (2009), Nature Immunology, 10 (8): 889-898). IgM is expressed on the surface of B cells (monomeric structure) as well as in a secreted form (pentameric structure) with very high avidity. IgM is involved in the elimination of pathogens in the early stages of B-cell-mediated (i.e. humoral) immunity before sufficient isotype-switching to IgG has occurred (Pier et al. (2004). Immunology, Infection, and Immunity, ASM Press; Geisberger et al. (2006), Immunology; 118(4):429-437).

Surprisingly, it has been demonstrated in accordance with the present invention that the ratio of expression levels of IgD versus IgM (also abbreviated herein as DvM or DvM score) in CLL patients correlates with clinical courses of this disease. The method of the invention now allows the treating physician to predict the clinical course of CLL with high reliability and consider appropriate treatment regimens, in particular at early such as non-symptomatic stages of the disease. The present invention confers significant advantages over the prior art that relies, inter alia, on the sequencing of the nucleic acid molecules encoding variable regions of immunoglobulins in order to provide an adequate prognosis. Sequencing of immunoglobulin genes is tedious, time-consuming and expensive and usually cannot be performed in-house in clinical laboratories. Due to the low costs and simple and fast procedure, the present invention can be performed in-house in clinical laboratories using well established procedures, reagents and instrumentation. This allows for a more rapid determination of the prognosis and may accelerate the onset of therapy. Due to the high specificity and sensitivity of the antibodies used to determine IgD and IgM expression, the present invention may also speed up the diagnosis as such or the confirmation of an initial CLL diagnosis. Approximately 25% of CLL patients with mutated immunoglobulin genes who according to the prior art have a relatively good prognosis nonetheless develop an aggressive course of the disease. This unreliability persists, even when the expression of ZAP70 expression is determined, which is a common method for establishing a prognosis of CLL in the prior art. The present invention is able to detect these cases and thus greatly increases the reliability of prognosis.

The present invention supports the treating physician in deciding whether or not treatment should be initiated. In the latter case, it further helps the physician to decide on the intervals of examination and in the former case it may contribute to the decision on the kind of treatment, e.g. aggressive or mild regimens.

In a preferred embodiment of the method of the invention, a ratio of an expression level of IgD vs. IgM ≤ 1.15 is indicative of the patient developing an aggressive clinical course of CLL. This preferred embodiment corresponds to a method of prognosing whether a patient afflicted with chronic lymphocytic leukemia (CLL) develops an aggressive clinical course of CLL comprising determining the expression levels of IgD and IgM in a sample obtained from said patient wherein a ratio of an expression level of IgD vs. IgM ≤ 1.15 is indicative of the patient developing an aggressive clinical course of CLL. As a logical consequence, a value >0.87 for the reciprocal ratio, i.e. IgM versus IgD, is also indicative of an aggressive clinical course.

In a different preferred embodiment of the method of the invention, a ratio of an expression level of IgD vs. IgM > 1.15 is indicative of the patient developing an indolent clinical course of CLL. This preferred embodiment corresponds to a method of prognosing whether a patient afflicted with chronic lymphocytic leukemia (CLL) develops an indolent clinical course of CLL comprising determining the expression levels of IgD and IgM in a sample obtained from said patient wherein a ratio of an expression level of IgD vs. IgM > 1.15 is indicative of the patient developing an indolent clinical course of CLL.

As can be taken from the appended examples, samples of 65 patient afflicted with CLL were analysed and it could be established that the indicated borderline values (DvM scores) provide an increased and statistically significant certainty for these 65 patients as regards the clinical course of their disease. Whereas it cannot be excluded that other borderline values may be established for different patient groups, it is assumed that these DvM scores also hold true for such other patient groups. In addition, future data (i.e increased numbers of patients analysed with the method of the present invention) may result in a minute variation of this borderline value. As with any other comparable method, it is assumed that the closer the DvM score reaches said borderline value, the more advantageous it is to support the results by additional prognostic tests. The indicated borderline values were obtained with the procedures, reagents and instruments described in this specification. Using different procedures, reagents and/or instrumentation may result in a variation of said borderline value. Thus, it may be necessary to establish individual borderline values by the use of procedures and/or reagents suitable to standardize measurements. The skilled person knows how to establish such borderline or threshold values. For example, the expression levels of IgD and IgM could be measured by the method of interest simultaneously in at least 10, more preferably at least 75 and most preferably at least 100 patients and statistical methods, such as receiver operating characteristics, could be employed to establish the borderline value for the individual method.

In another preferred embodiment of the method of the invention, said patient is a patient in stage A according to the classification of Binet or in stage 0, I, or II according to the classification of Rai.

The Rai and/or Binet clinical staging is frequently used for prognosing CLL progression. According to the classification of Rai, stage 0 is characterized by an absolute lymphocytosis (15,000 or more lymphocytes per µl in peripheral blood, with 40% or more lymphocytes found in the bone marrow). In addition, at stage I, lymph nodes are enlarged. At stage II lymphocytosis is accompanied by an enlarged spleen or liver or both, while at stage III lymphocytosis comes along with anemia (haemoglobin below 11g/100ml or hematocrit below 33%), irrespective of whether nodes, spleen or liver are enlarged or not. Finally, at stage IV lymphocytosis is combined with thrombocytopenia (number of platelets less than 100,000 per µl, irrespective of the presence of anemia or organomegaly (Rai, K.R. et al. (1975) Blood 46: 219-234). According to the Binet classification, stage A is characterized by lymphocytosis with a seizure of less than 3 lymph node regions (neck, armpit, groin, liver or spleen) and the absence of anemia or thrombocytopenia (corresponding to Rai stages 0, I, and II). Stage B is characterized by lymphocytosis with a seizure of more than 3 lymph node regions (neck, armpit, groin, liver or spleen) and the absence of anemia or thrombocytopenia (corresponding to Rai stages I and II). Stage C is characterized by lymphocytosis with anemia and/or thrombocytopenia regardless of the number of areas of lymphoid enlargement (corresponding to Rai stages III and IV) (Binet, J.L. et al. (1981) Cancer 48: 198-206).

The symptoms of CLL are variable and can range from basically asymptomatic courses, with patients presenting with general weakness only, through moderate symptoms such as weight loss, fever, night sweats, and pressure symptoms from enlarges nodes, spleen, or liver to severe symptoms such as shortness of breath, easy bruising, recurrent infections, and bleeding. The symptoms do roughly but not strictly correspond to the clinical staging according to Rai or Binet.

Performing the method of the invention at early stages of the disease allows the physician to take counter-measures that may prolong the lifetime of the patient. Before the background that the disease starts developing at an average patient age beyond 50 years and most often at an age of 65 to 70 years, such counter-measures may result in CLL not being fatal for the patients. In addition, symptoms of CLL that make the patient suffer and that usually emerge only in later stages of the disease may be avoided or at least postponed. Alternatively, the method of the invention may be put into practice at non-symptomatic stages of the disease. The method of the invention may allow the physician to decide whether and when counter-measures should be taken. Depending on the course of the disease predicted by the method of the invention, it may be beneficial to start treatment before symptoms arise. In addition, it may allow the physician to decide on the kind of counter-measures that should be taken. A course of the disease that is predicted by the method of the invention to be rather indolent may allow for a rather mild treatment regimen whereas a predicted aggressive course may ask for an aggressive treatment regimen.

In a different preferred embodiment of the method of the invention, the patient is a patient with leukemia cells that express mutated B cell receptors.

The term "mutated B cell receptors" refers to those receptors which carry mutations in the nucleic acid sequences encoding the variable regions of the B cell receptors, preferably in the complementarity determining regions (CDRs) and most preferred in CDR 3. These mutations arise from somatic hypermutation in the course of affinity maturation during a germinal centre reaction and can be detected by sequencing of the variable region genes. A B-CLL is considered to be mutated if the sequence of the immunoglobulin heavy chain variable region (IgV_{H}) expressed on the diseased cells differs from the corresponding germ line sequence in more than 2%.

In an alternative preferred embodiment of the method of the invention, the patient is a patient with leukemia cells that express unmutated B cell receptors.

The term "unmutated B cell receptors" refers to those receptors which do not carry mutations in the nucleic acid sequences encoding the variable regions of the B cell receptors.

As discussed herein above, patients carrying such mutations have been correlated in the art with a better prognosis of CLL than patients not carrying such mutations. If the mutational status of the patient is known, then the method of the invention is expected to confirm such an aggressive or indolent course of the disease. Conversely, if the mutational status of the patient is unknown, then prognosis obtained with the method of the invention is expected to allow conclusions with regard to the mutational status of the invention: If an aggressive course of the disease is predicted, then a high likelihood for the patient carrying non-mutated nucleic acid sequences exists. On the other hand, a DvM score of > 1.15 obtained with the method of the invention is indicative of a patient carrying mutations in the mentioned nucleic acid molecules.

In one embodiment, the expression is measured on the transcriptional level.

Methods for measuring the levels of factors, such as immunoglobulins, on the transcriptional, i.e. RNA level are known in the art and include separation by gel electrophoresis and Northern blotting, reverse transcription (quantitative) polymerase chain reaction (RT-PCR or RT-qPCR), fluorescence in situ hybridization (FISH), biochip technologies, such as DNA-microarrays, individually tagging single mRNA molecules with fluorescent barcodes (nanostrings) or serial analysis of gene expression (SAGE). Detailed descriptions and protocols of the above mentioned methods are well-known in the art and can be found, for example, in Kevil, C. G. et al., Biochem. Biophys. Res. Commun. 238, 277-279 (1997) for Northern blotting, Page, R. B. and Stromberg, A. J., Scientific World Journal 11, 1383-1393 (2011) for RT-PCR, Guenin, S. et al., J. Exp. Bot. 60, 487-493 (2009) for RT-qPCR, Sarrate, Z. et al., Fertil. Steril. 93, 1892-1902 (2010) for FISH, Schema, M. et al., Science 270, 467-470 (1995) for DNA microarrays, Geiss, G. K. et al., Nature Biotechnology 26, 317-325 (2008) for quantitative nanostring technologies and Velculescu, V. E. et al., Science 270, 484-487 (1995) for SAGE.

Preferably, expression on the transcriptional level is determined on a micro-array. Micro-arrays are biochips that have specific nucleic acids immobilised on their surfaces to which complementary nucleic acids can hybridize, followed by detection of hybridization. Preferably, the expression of IgM and IgD on the transcriptional level is determined by a micro-array in parallel to that of other prognostic markers for CLL, such as ZAP70, CD38 or CD23, or of particular microRNAs such as miR15a-miR16-1, miR34b/miR34c, miR21 or miR181b. Subsequently, the prognosis regarding the course of CLL can again be based on a DvM score or, especially in cases where this score is close to the borderline value established for this method, on a combination of the DvM score with the results obtained for the other prognostic markers.

In another preferred embodiment of the method of the invention, expression is measured on the translational level.

Also, methods for measuring the levels of immunoglobulins on the translational, i.e. protein level are known in the art and include SDS polyacrylamide gel electrophoresis (SDS-PAGE) and Western blotting, microscopy based techniques, for example immunofluorescence or histological staining, enzyme-linked immunosorbent assay (ELISA), radioimmunoassay (RIA), mass spectrometry based technologies, for example isobaric tags for relative and absolute quantification (iTRAQ) or stable isotope labelling with amino acids in cell culture (SILAC), biochip technologies, such as protein- or peptide microarrays or fluorescence activated cell sorting (FACS). Detailed descriptions and protocols of the above mentioned methods are well-known in the art and can be found, for example, in Sambrook, J. and Russell, D.W., Molecular Cloning: A Laboratory Manual, CSHL Press (1989) for SDS-PAGE and Western blotting, Pohla-Gubo, G. and Hintner, H., Dermatol. Clin. 3, 365-372 (2011) for immunofluoresce, Lequin, R., Clin. Chem. 51, 2415-2418 (2005) for ELISA, Acebedo, G. et al., Biochem. Biophys. Res. Commun. 65, 449-456 (1975) for RIA or Petriz, B.A. et al., J. Cell Physiol. 227, 885-898 (2011) for mass spectrometry based methods.

In case that expression is measured on the translational level it is preferred that expression is measured by FACS analysis.

FACS (fluorescence activated cell sorting) analysis is a specific subcategory of flow cytometric methods and well established in the art. FACS provides a method for sorting a heterogeneous mixture of cells into subgroups, one cell at a time, based upon the specific light scattering and fluorescence characteristics of each cell. The term "FACS analysis" also includes procedures in which cells are not sorted but only analysed for the presence or amount of a given characteristic such as e.g. fluorescence. Fluorescence characteristics of a cell might be manipulated by antibody labels, expression of free or fused fluorescent proteins like GFP or RFP or by staining cell structures with specific agents, for example, propidium iodide. The use of differentially labelled antibodies allows for the presence and/or amount of different factors to be determined within the same sample and at the same time. Alternatively, the addition of standardised reagents to different samples allows the standardisation and comparison of absolute fluorescence values measured in these samples. Detailed protocols for FACS analysis are well-known in the art and can be found, for example, in Shapiro, H. M., Practical Flow Cytometry, 4th Edition, John Wiley & Sons (2003). Appropriate sorting machines can be purchased, for example, from Becton Dickinson and Company (Franklin Lakes, U.S.A.).

The expression of IgD and IgM can be detected by one or more poly- or monoclonal antibodies, each carrying a detectable label such as a fluorescent label. As described above, the ratio of the detected fluorescence intensities for IgD and IgM serves as a predictive value for the prognosis of disease progression. It is further preferred that detection of IgD and IgM expression is effected in the same tube, i.e. performing a double staining for IgD and IgM using two different detection labels.
Alternatively, labelling of IgD is performed in one tube and labelling of IgM is performed in a different tube, both containing the identical patient's sample. In this case, absolute fluorescence intensities are measured and are standardized using appropriate reagents and procedures. Such reagents and procedures are known by the art and are commercially available (e.g. QuantiBrite series (Becton-Dickinson) or QSC microspheres (Bangs Laboratories)) (Rossmann E.D. et al. (2007) Cytometry B Clin. Cytom. 72: 450-457; NCCLS. Fluorescence Calibration and Quantitative Measurement of Fluorescence Intensity; Approved Guideline. NCCLS document I/LA24-A [ISBN 1-56238-543-7]. NCCLS, 940 West Valley Road, Suite1400, Wayne, Pennsylvania 19087-1898 USA, 2004.).

It is further preferred that the sample is a sample from peripheral blood, bone marrow, lymph nodes, liver or spleen.

Appropriate steps for preparing samples suitable for analysis in accordance with a diagnostic or prognostic method, such as the method of the present invention, are well known in the art. This can include, for example, centrifugation of the sample. Especially in the case of samples derived from peripheral blood, the addition of appropriate anticoagulants (e.g. EDTA and/or Heparin) can also be comprised in the preparation process. Further, sample processing can comprise the lysis of cells such as, for example, erythrocytes or the isolation of specific cell types or subcellular components and/or the transfer of cells or other components of the original sample into buffers appropriate for analysis. In addition, the incubation with labelled, e.g. fluorescently labelled, agents such as e.g. antibodies against IgM, IgD and/or other proteins can also constitute part of the sample preparation process.

Generally, it is advantageous that the determination of the ratio of the expression labels is effected or assisted by mathematical or statistical methods. As discussed herein above, the use of different procedures may result in a variation of the threshold value so that it may be necessary to establish individual borderline values. In this regard, it is preferred, that the determination of the threshold for the ratio of the expression level is assisted by receiver operator characteristic (ROC). A ROC curve can be construed by establishing sensitivity and specificity for the different test values and by plotting 1-specificity on the x-axis and sensitivity on the y-axis. Specificity is a ratio between subjects with a true-negative test result and the total number of subjects with an indolent course of CLL. Sensitivity is a ratio between the number of subjects with a true-positive test result and the total number of subjects with an aggressive course of CLL. The optimal threshold value results from the point closest to the upper left corner of the graph of the ROC curve, i.e. the point where (1 - sensitivity)² + (1 - specificity)² reaches a minimum.

Finally, the method of the invention in another preferred embodiment further comprises sequencing of the variable regions of immunoglobulin genes or parts thereof to determine their mutation status, determination of ZAP70 expression, determination of chromosomal aberrations (e.g. del(17p), del(11q), trisomy 12), determination of the expression of surface markers such as CD38, CD23, F(c)-receptor-like proteins (FCRLs), in particular FCRL2 or/and determination of serum markers such as lactate dehydrogenase, CD49d, beta-2-microglobulin, thymidine kinase, or/and sCD23. These analyses are established in the art as methods for prognosing the clinical course of CLL. Whereas high expression of FCRL2 is thought to indicate an indolent course of CLL, trisomy 12 is typically considered to be associated with an intermediary to poor clinical course and chromosomal deletions comprising the genes *ATM* (del11(q)) or *TP53* (del17(p)) are used as indicators of a poor prognosis. Similarly, high expression levels of ZAP70, CD38 or CD49d as well as high serum levels of beta-2-microglobulin, lactate dehydrogenase, sCD23 or thymidine kinase are also employed as indicators of a poor prognosis (Crespo M. et al., N Engl J Med. 348:1764-1775 (2003); Delgado J. et al., Br J Haematol. 145:801-805 (2009); Konoplev SN. et al., Am J Clin Pathol. 134(3):472-7 (2010); Molica S. et al., Haematologica. 81(5):428-33 (1996); Zucchetto A. et al., Leukemia. 20(3):523-5 (2006)). Thus, these additional measures can be taken to bolster the statistical significance of the prognosis obtained by the method of the invention. The various alternatives mentioned in this preferred embodiment of the invention refer to methodology known *per se* in the art that is used in the prognosis of CLL. Since these measures are, however, accompanied by the disadvantages associated with prior art methods, they are usually not combined with the other embodiments of the invention. However, since the advantages associated with the present invention can overcome some of the disadvantages associated with the methods of the prior art, a combination is envisioned especially in cases where a confirmation of the prognosis is desired.

The figures show:

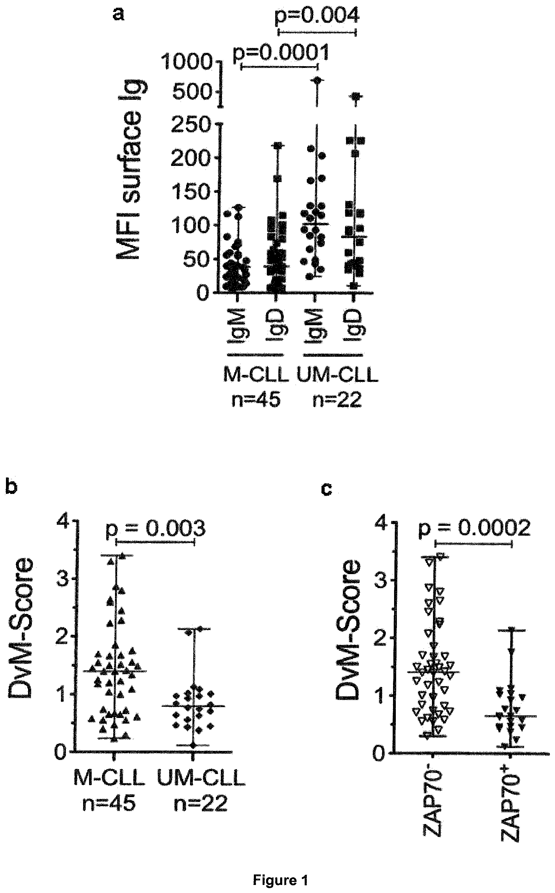

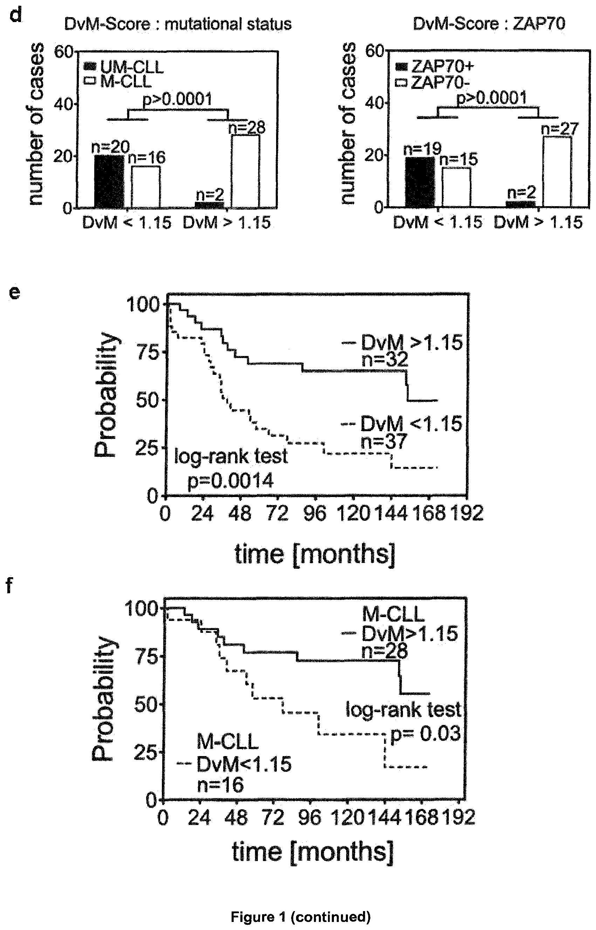

**Figure 1** Surface expression of IgD and IgM serves as a diagnostic marker.
   (a) MFI values for surface IgM (circles) and IgD (squares) of randomly chosen CLL cases (n=65). Horizontal line represents median values; vertical lines indicate the range. P-values were calculated using Mann-Whitney-Test. (b,c) Correlation of DvM-Scores with (b) mutational status of the BCR and (c) ZAP70 expression in CLL cells. Median values are represented by a horizontal line; vertical lines indicate the range. P-values were calculated using Mann-Whitney-Test. (d) Correlation of the DvM-Score and the indicated prognostic parameters in CLL as determined by Fisher-Exact-Tests performed using a DvM threshold of 1.15. (e) Kaplan-Mayer curve representing the correlation between DvM-Score and time to first treatment for CLL-cases using a DvM threshold of 1.15. (f) Kaplan-Mayer curve representing the correlation between DvM-Score and time to first treatment for M-CLL-cases. P values were calculated using log-rank test.

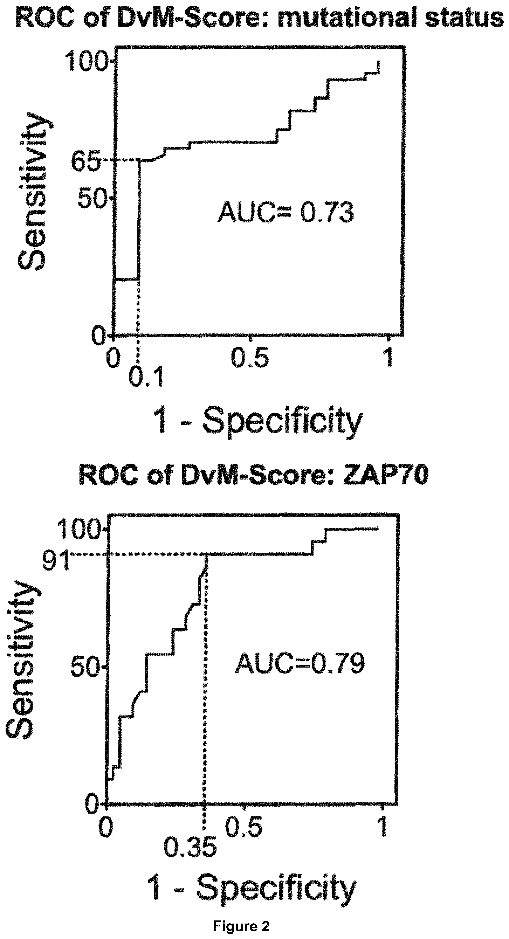

**Figure 2** Receiver operating characteristic for the correlation between DvM-Score, mutation status and ZAP70 expression.
   ROC curves calculated for DvM-Score in correlation to the mutational status of the BCR and to ZAP70 expression. Dashed lines represent the values for the chosen threshold of 1.15. AUC indicates the area under the curve which is an index for the accuracy of the test.

The examples illustrate the invention.

### Example 1: Materials and Methods

### Lymphoma material and clinical data

B-CLL samples were obtained from peripheral blood of patients at University Medical Center Freiburg. Data for IGVH status, ZAP70 expression and time to first treatment were taken from the clinical records. The local ethic committee had approved patients sampling and all patients gave informed consent.

### Flow cytometry

Human CLL samples were stained with mouse anti-human CD19 (BD), mouse anti human CD5 (BD), mouse anti-human IgM (Southern Biotech), and mouse anti-human IgD (Southern Biotech).

### Statistical analysis

For statistical analysis Graph Pad Prism software was used. All data sets were tested for normal distribution performing D'Agostino & Pearson omnibus normality test (alpha=0.05).

### Example 2: The clinical course of CLL correlates with IgM expression

To investigate whether the expression levels of IgM and IgD on CLL cells are associated with disease prognosis, 65 randomly chosen CD19/CD5-positive CLL samples were stained for both surface IgM and IgD. In agreement with previous reports, expression levels of both IgM and IgD were significantly higher in UM-CLL cases as compared to M-CLL cases (Figure 1a) (Vilpo, J., et al., Eur J Haematol 70, 53-59 (2003)). Using mean fluorescent intensities we then calculated the ratio of IgD versus IgM expression (herein also referred to as DvM-Score) for both subsets. UM-CLL cases often expressed less IgD than IgM resulting in DvM-Score below 1, whereas the majority of M-CLL cases had a DvM-Score of about 1.5 due to higher levels of IgD as compared to IgM, which also correlated with ZAP70 expression (Figure 1b, c). By performing receiver operating characteristic, a threshold of 1.15 was determined for diagnosis (Figure 2). Using this threshold, a strong correlation between the DvM-Score and known diagnostic markers as mutational status and ZAP70 (Figure 1d) was found. Together, this suggests that a DvM-Score >1.15 is associated with a more indolent course of disease, whereas a DvM-Score ≤1.15 is an indicator for an aggressive form of CLL. In agreement with this, cases with a DvM-Score >1.15 had a median time to first treatment (TTFT) of 218 month, while cases with a DvM-Score ≤1.15 had a median TTFT of 40 month (Figure 1e). Importantly, the DvM-Score identified a group of M-CLLs with a poor prognosis similar to that of UM-CLL (Figure 1f) suggesting that the DvM-Score is a proper indicator for the clinical course of CLL. Thus, the difference between IgM and IgD now allows determining the prognosis of CLL cases based on the expression of IgD versus IgM. Interestingly, the presence of B cells that express IgD and no IgM resulted in a significant attenuation of CLL development and reduced the expansion of IgM-expressing CLL B cells in TCL1-transgenic mice. Whereas the applicants do not wish to be bound by any scientific theory, this indicates that the expansion of CLL B cells is controlled by co-existing non-transformed B cells, which may be of importance when deciding which and when counter-measures are to be taken. In summary, the invention suggests that investigating the expression profiles of IgD and IgM may not only provide a simple method to assess the individual prognosis of a CLL patient but may also lead to the development of novel therapeutic approaches.

## Claims

1. A method of prognosing whether a patient afflicted with B-cell chronic lymphocytic leukemia (B-CLL) with leukemia cells that express mutated B cell receptors develops an aggressive or an indolent clinical course of B-CLL comprising determining the expression levels of IgD and IgM in a sample obtained from the peripheral blood of said patient and employing the ratio of IgD and IgM expression levels as an indicator for predicting whether said patient develops an aggressive or an indolent clinical course of B-CLL, wherein a ratio of an expression level of IgD vs. IgM ≤ 1.15 is indicative of the patient developing an aggressive clinical course of B-CLL and wherein a ratio of an expression level of IgD vs. IgM > 1.15 is indicative of the patient developing an indolent clinical course of B-CLL.

2. The method of claim 1 wherein said patient is a patient in stadium A according to the classification of Binet or stage 0, I, or II according to the classification of Rai.

3. The method of claim 1 or 2 wherein expression is measured on the transcriptional level.

4. The method of claim 3 wherein expression on the transcriptional level is measured on microarrays/ biochips.

5. The method of claim 1 or 2 wherein expression is measured on the translational level.

6. The method of claim 5 wherein expression is measured by FACS analysis.

7. The method of any one of claims 1 to 6 wherein the determination of the threshold for the ratio of the expression level is assisted by receiver operator characteristic.

8. The method of any one of claims 1 to 7 further comprising sequencing of the variable regions of immunoglobulin genes or parts thereof to determine their mutation status, determination of ZAP-70 expression, determination of chromosomal aberrations (e.g. del(17p), del(11q), trisomy 12), determination of the expression of surface markers such as CD38, CD23, F(c)-receptor-like proteins (FCRLs), in particular FCRL2 or/and determination of serum markers such as lactate dehydrogenase, CD49d, beta-2-microglobulin, thymidine kinase, or/and sCD23.

## Patentansprüche

1. Verfahren zur Vorhersage, ob ein Patient, der an B-Zellen chronischer lymphatischer Leukämie (B-CLL) mit Leukämiezellen leidet, die mutierte B-Zell-Rezeptoren exprimieren, einen aggressiven oder einen indolenten klinischen Verlauf von B-CLL entwickelt, umfassend das Bestimmen der Expressionswerte von IgD und IgM in einer Probe, die aus dem peripheren Blut des Patienten erhalten wurde, und das Verwenden des Verhältnisses der IgD- und IgM-Expressionswerte als Indikator für die Vorhersage, ob der Patient einen aggressiven oder einen indolenten klinischen Verlauf von B-CLL entwickelt, wobei ein Verhältnis der Expressionswerte von IgD vs. IgD ≤ 1,15 indikativ dafür ist, dass der Patient einen aggressiven klinischen Verlauf von B-CLL entwickelt, und wobei ein Verhältnis der Expressionswerte von IgD vs. IgM > 1,15 indikativ dafür ist, dass der Patient einen indolenten klinischen Verlauf von B-CLL entwickelt.

2. Verfahren nach Anspruch 1, wobei der Patient ein Patient im Stadion A gemäß der Klassifizierung von Binet oder der Stufe 0, I oder II gemäß der Klassifizierung von Rai ist.

3. Verfahren nach Anspruch 1 oder 2, wobei die Expression auf der Transkriptionsebene gemessen wird.

4. Verfahren nach Anspruch 3, wobei die Expression auf der Transkriptionsebene mittels Microarrays/Biochips gemessen wird.

5. Verfahren nach Anspruch 1 oder 2, wobei die Expression auf der Translationsebene gemessen wird.

6. Verfahren nach Anspruch 5, wobei die Expression mittels FACS Analyse gemessen wird.

7. Verfahren nach einem der Ansprüche 1 bis 6, wobei das Bestimmen des Schwellenwertes für das Verhältnis der Expressionswerte durch die Operationscharakteristik eines Beobachters unterstützt wird.

8. Verfahren nach einem der Ansprüche 1 bis 7, ferner umfassend das Sequenzieren der variablen Regionen von Immunglobulingenen oder Teilen derselben zum Bestimmen ihres Mutationsstatus, das Bestimmen der ZAP-70 Expression, das Bestimmen der chromosomalen Aberrationen (z.B. del(17p), del(11q), Trisomie 12), das Bestimmen der Expression von Oberflächenmarkern wie CD38, CD23, F(c)-Rezeptor-ähnlichen Proteinen (FCRLs), insbesondere FCRL2 oder/und das Bestimmen von Serummarkern wie Laktatdehydrogenase, CD49d, Beta-2-Microglobulin, Thymidinkinase, oder/und sCD23.

## Revendications

1. Une méthode pour pronostiquer si un patient atteint d'une leucémie lymphoïde chronique de type B (B-LLC) avec des cellules leucémiques exprimant des récepteurs de cellules B mutés développera une forme clinique grave ou bégnine de B-LLC comprenant la détermination des taux d'expression des IgD et des IgM dans un échantillon de sang périphérique du dit patient et l'utilisation du ratio du taux d'expression d'IgD par rapport à celui des IgM comme marqueur de prédiction chez le dit patient d'évolution clinique grave ou bégnine de B-LLC, dans laquelle un ratio du taux d'expression des IgD par rapport aux IgM ≤ 1,15 est un marqueur de développement chez le patient d'une forme clinique grave de B-LLC et dans laquelle un ratio du taux d'expression des IgD par rapport aux IgM > 1,15 est un marqueur chez le patient de développement d'une forme clinique bégnine de B-LLC.

2. La méthode selon la revendication 1, dans laquelle le dit patient est un patient au stade A selon la classification de Binet ou au stade 0, I, ou II selon la classification de RAI.

3. La méthode selon les revendications 1 ou 2, dans laquelle l'expression est mesurée au niveau de la transcription.

4. La méthode selon la revendication 3, dans laquelle l'expression au niveau de la transcription est mesurée par puce à ADN / biopuces.

5. La méthode selon les revendications 1 ou 2, dans laquelle l'expression est mesurée au niveau de la translation.

6. La méthode selon la revendication 5, dans laquelle l'expression est mesurée par cytométrie de flux.

7. La méthode selon une des revendications 1 à 6, dans laquelle la détermination du seuil pour le ratio des taux d'expression est assistée par la caractéristique de fonctionnement du récepteur.

8. La méthode selon une des revendications 1 à 7 comprenant de plus le séquençage des régions variables des gènes d'immunoglobuline ou de parties de ceux-ci pour déterminer leur statut mutationnel, la détermination de l'expression de ZAP-70, la détermination des aberrations chromosomiques (par ex. del(17p), del(11q), trisomie 12), la détermination de l'expression des marqueurs de surface tels que CD38, CD23, les protéines analogues au récepteur F(c) (FCRLs), en particulier FCRL2 ou/et la détermination des marqueurs sériques tels que la lactate déshydrogénase, CD49d, la béta-2 microglobuline, la thymidine kinase, ou/et sCD23.
